(19) Europäisches Patentamt
European Patent Office
Office européen des brevets

(11) **EP 1 838 245 B1**

## (12) EUROPEAN PATENT SPECIFICATION

(45) Date of publication and mention of the grant of the patent:
**02.03.2011 Bulletin 2011/09**

(21) Application number: **05852535.3**

(22) Date of filing: **01.12.2005**

(51) Int Cl.:
***A61F 2/16*** (2006.01)

(86) International application number:
**PCT/US2005/043318**

(87) International publication number:
**WO 2006/060477 (08.06.2006 Gazette 2006/23)**

(54) **CONTRAST-ENHANCING ASPHERIC INTRAOCULAR LENS**

KONTRASTVERSTÄRKENDE ASPHÄRISCHE INTRAOKULARLINSE

LENTILLE INTRAOCULAIRE ASPHERIQUE AMELIORANT LE CONTRASTE

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.12.2004 US 728**

(43) Date of publication of application:
**03.10.2007 Bulletin 2007/40**

(73) Proprietor: **Alcon, Inc.**
**6331 Hünenberg (CH)**

(72) Inventor: **SIMPSON, Michael J.**
**Arlington, Texas 76012 (US)**

(74) Representative: **Hanna, Peter William Derek et al**
**Hanna Moore & Curley**
**13 Lower Lad Lane**
**Dublin 2 (IE)**

(56) References cited:
**EP-A- 0 742 466    WO-A-01/89424**
**WO-A-2004/034129    US-A- 4 504 982**

**Description**

TECHNICAL FIELD OF THE INVENTION

The present invention generally relates to intraocular lenses (IOL) and, more particularly, to such lenses that provide enhanced vision for large pupil sizes.

An intraocular lens is routinely implanted in a patient's eye during cataract surgery to compensate for the lost optical power when the natural lens is removed. In other applications, an intraocular lens can be implanted in a patient's eye, which retains its natural lens, so as to provide an optical power for correcting a refractive error of the natural eye. The aberrations of the eye, and in particular those of the cornea, are typically ignored in designing conventional intraocular lenses. Hence, patients having such lenses can suffer from a degraded image quality, especially at low light levels and large pupil sizes.

Intraocular lenses that compensate for corneal aberrations are also known. Typically, such aspheric intraocular lenses are designed to counteract the asphericity of the patient's cornea by greatly reducing, or eliminating all together, the overall aberrations of the eye. Although intraocular lenses fabricated based on these techniques may provide a better image contrast, they generally result in a decrease in a patient's depth of field. The state of the art is represented by US-A-4,504,982 (Burk et at) and WO 01/89424 A (Pharmacia Groningen B.V. et al).

BRIEF SUMMARY OF THE INVENTION

The present invention is generally directed to intraocular lenses that can provide a balance between image contrast and depth of field, upon implantation in a patient's eye, so as to afford the patient improved vision, especially under conditions where the pupil of the eye is large. More particularly, an intraocular lens of the invention can exhibit a selected degree of asphericity at one or more refractive surfaces such that the aberrations of the lens combine with those of the eye in a manner that would provide the patient not only with a useful image contrast, but also with a depth of field within an acceptable range, especially for large pupil sizes.

In one aspect, the present invention provides an intraocular lens (IOL) that includes an optic having an anterior refractive surface and a posterior refractive surface, which cooperatively provide a selected optical power, e.g., an optical power in a range of about zero to about 40 Diopters (D) or more, typically in a range of about 18 to about 26 Diopters. One or both of these surfaces are characterized by an aspheric profile for controlling the aberrations of an eye in which the IOL is implanted so as to provide the patient with an image contrast, as characterized by a peak modulation transfer function (MTF), of at least about 0.25 at a spatial frequency of about 50 line pairs per millimeter (lp/mm) and a depth of field of at least about 0.75 Diopters (D). For example, the implanted lens can provide the patient with an MTF in a range of about 0.25 to about 0.4 and a depth of field in a range of about 0.75 to about 1.5 Diopters. The aspherical lenses of the present invention can control the aberrations of the eye of a pseudophakic patient, i.e., a patient having the IOL as a replacement for a natural lens. Alternatively, such lenses can control the aberrations of the eye of a phakic patient, i.e., a patient having the IOL in addition to the natural lens.

As is known to those skilled in the ophthalmic art, a modulation transfer function (MTF) provides a quantitative measure of image contrast exhibited by an optical system, e.g., a system formed of an IOL and the cornea or an optical system formed of an IOL, the cornea and the natural lens, as discussed in more detail below. Further, the terms "depth of field" and "depth of focus," which are herein used interchangeably, are well known in the context of a lens and readily understood by those skilled in the art. To the extent that a quantitative measurement is needed to describe the present invention, the term "depth of field" or "depth of focus" as used herein, can be calculated and/or measured by an amount of defocus associated with the optical system at which a through-focus modulation transfer function (MTF) of the system calculated and/or measured with an aperture, e.g., a pupil size, of about 4.5 mm and monochromatic green light, e.g., light having a wavelength of about 550 nm, exhibits a contrast of at least about 0.05 at a spatial frequency of about 50 line pairs per millimeter (lp/mm).

In a related aspect, the aspheric profile of the anterior surface or the posterior surface, or both, can control the aberrations of an eye in which the IOL is implanted such that the combined lens and cornea would exhibit a peak modulation transfer function contrast of at least about 0.25 at a spatial frequency of about 50 lp/mm and a depth of field of at least about 0.75 Diopters for pupil diameters in a range of about 4.5 mm to about 5 mm and for monochromatic light at a wavelength of about 550 nm. For example, the peak modulation transfer function can be calculated in a model eye, as discussed in more detail below.

An IOL according to this invention can be fabricated preferably by employing a deformable biocompatible material, such as acrylic, silicone, or hydrogel polymeric materials and the like that allow the lens body to be folded for insertion into the eye. For example, the optic can be formed of a copolymer of acrylate and methacrylate. For illustrative examples of such copolymer compositions, see for example, U.S. Patent No. 5,922,821 entitled "Ophthalmic Lens Polymers" issued to Lebouef *et al.* on July 13, 1999 and U.S. Patent No. 6,353,069 entitled "High Refractive Index Ophthalmic Device Materials" issued to Freeman *et al.* on March 5, 2002. In other embodiments, rigid biocompatible materials, such as polymethyl methacrylate (PMMA) can be employed.

In some embodiments, the aspherical profile of one of the surfaces can exhibit a selected deviation from a putative spherical profile having a radius of curvature of $R_1$ that substantially coincides with the aspherical profile at small radial distance from an optical axis of the lens, while the other surface can have a spherical profile having a radius of curvature $R_2$. Alternatively, the other surface can also have an aspherical profile exhibiting deviations from a respective putative spherical profile having a radius of curvature of $R_2$. The radii $R_1$ and $R_2$ are selected such that the lens would exhibit a desired optical power. In addition, if needed, $R_1$ and $R_2$ can be chosen to impart a selected shape factor ($X$) to the lens, which is generally defined by the following relation:

$$X = \frac{R_2 + R_1}{R_2 - R_1} .$$

In a related aspect, at least one refractive surface of the IOL has an aspheric portion for controlling average aberrations exhibited by the eyes of a selected patient group such that upon implantation of the lens in a patient's eye the combined lens and cornea would exhibit a peak modulation transfer function (MTF) contrast of at least about 0.25 for monochromatic light having a wavelength of 550 nm and depth of field of at least about 0.75 Diopters. The MTF and the depth of field can be calculated or measured, for example, for a spatial frequency of about 50 line pairs per millimeter and for a pupil size of about 4.5 mm.

In another aspect, the profile of the aspheric surface can be characterized by the following relation:

$$z = \frac{CR^2}{1 + \sqrt{1 - (1+Q)C^2 R^2}} + AR^4 + BR^6 + \text{ higher order terms} ,$$

wherein

$z$ denotes a sag of the surface parallel to an axis (z) perpendicular to the surface,
$C$ denotes a curvature at the vertex of the surface,
$Q$ denotes a conic coefficient,
$R$ denotes a radial position on the surface,
$A$ denotes a fourth order deformation coefficient, and
$B$ denotes a sixth order deformation coefficient. Distance units are given herein in millimeters. For example, the curvature constant is given in units of inverse millimeter, while $A$ is given in units of $\frac{1}{(\text{mm})^3}$ and $B$ is given in units of $\frac{1}{(\text{mm})^5}$ .

The curvature constant $C$ can be chosen based on a desired optical power of the lens, and the aspherical coefficients $Q, A,$ and B, as well as the higher order terms where applicable, can be chosen so as to impart a selected degree of asphericity to the surface. As discussed in more detail below, the choice of the aspherical coefficients can generally depend on the material from which the lens is fabricated, the shape factor of the lens, and the aberrations of the eye for which the lens is intended. For example, the conic constant for a biconvex lens of average power (e.g., 21 Diopters) formed of an acrylic polymer can be in a range of about 0 (zero) to about -100 (minus 100), or in a range of -10 to about -50, or in a range of about -15 to about -25 and the higher order deformation coefficients $A$ and $B$ can be, respectively, in a range of about $-1 \times 10^{-3}$ (minus 0.001) to about $1 \times 10^{-3}$ (plus 0.001) and in a range of about $-1 \times 10^{-4}$ (minus 0.0001) to about $1 \times 10^{-4}$ (plus 0.0001). Further, in many embodiments, the curvature coefficient (C) can be in a range of about 0.0125 to about 0.12, or in a range of about 0.025 to about 0.1 (the curvature can be positive or negative corresponding to convex or concave surfaces, respectively).

In another aspect, the invention provides a method of designing an intraocular lens having an anterior and a posterior refractive surface that includes deriving a model average of aberrations of the eye based on wavefront measurements of aberrations exhibited by the eyes of a selected patient population (alternatively the aberrations of an individual patient for whom the lens is intended can be employed), and adjusting asphericity of at least one of the refractive surfaces for controlling the average aberrations such that a patient in which the lens is implanted would exhibit an image contrast characterized by a peak modulation transfer function (MTF) contrast of at least about 0.25 and a depth of field of at least about 0.75 D.

## BRIEF DESCRIPTION OF THE SEVERAL VIEWS OF THE DRAWINGS

FIGURE 1A schematically depicts an intraocular lens according to one embodiment of the invention having an anterior surface exhibiting an aspherical profile;

FIGURE 1B schematically illustrates a sag profile of the aspherical anterior surface of the IOL of FIGURE 1A exhibiting a selected deviation from a putative spherical profile;

FIGURE 1C schematically illustrates a sag profile of the spherical posterior surface of the IOL of FIGURE 1A;

FIGURE 2 schematically depicts a phakic eye having an IOL according to one embodiment of the invention in addition to the natural lens;

FIGURE 3A is a graph illustrating a theoretical modulation transfer function (MTF) calculated for the combined system of an eye having a spherical cornea and an IOL having spherical refractive surfaces;

FIGURE 3B is a graph illustrating a theoretical modulation transfer function (MTF) calculated for the combined system of an eye having a spherical cornea and an IOL according to one embodiment of the invention having an aspherical surface;

FIGURE 4A is graph illustrating a theoretical modulation transfer function (MTF) calculated for the combined system of an eye exhibiting corneal spherical aberration and an IOL having a spherical profile;

FIGURE 4B is a graph illustrating a theoretical modulation transfer function (MTF) calculated for the combined system of an eye exhibiting severe corneal flattening and an IOL according to one embodiment of the invention having an aspherical surface for controlling aberrations caused by the cornea;

FIGURE 5 is a graph illustrating a theoretical modulation transfer function (MTF) calculated for the combined system of an eye exhibiting an average corneal aberration and an IOL having spherical surfaces;

FIGURE 6 depicts three graphs depicting theoretically calculated peak modulation transfer function contrasts and depth of fields for various eye conditions with spherical IOLs and aspherical IOLs according to the teachings of the invention;

FIGURE 7A schematically depicts an exaggerated aspherical profile along one surface direction of a toric surface of an IOL according to one embodiment of the invention relative to a putative spherical profile, and

FIGURE 7B schematically depicts an exaggerated aspherical profile along another direction of the toric surface associated with the profile shown in FIGURE 7A relative to a putative spherical profile.

## DETAILED DESCRIPTION OF THE INVENTION

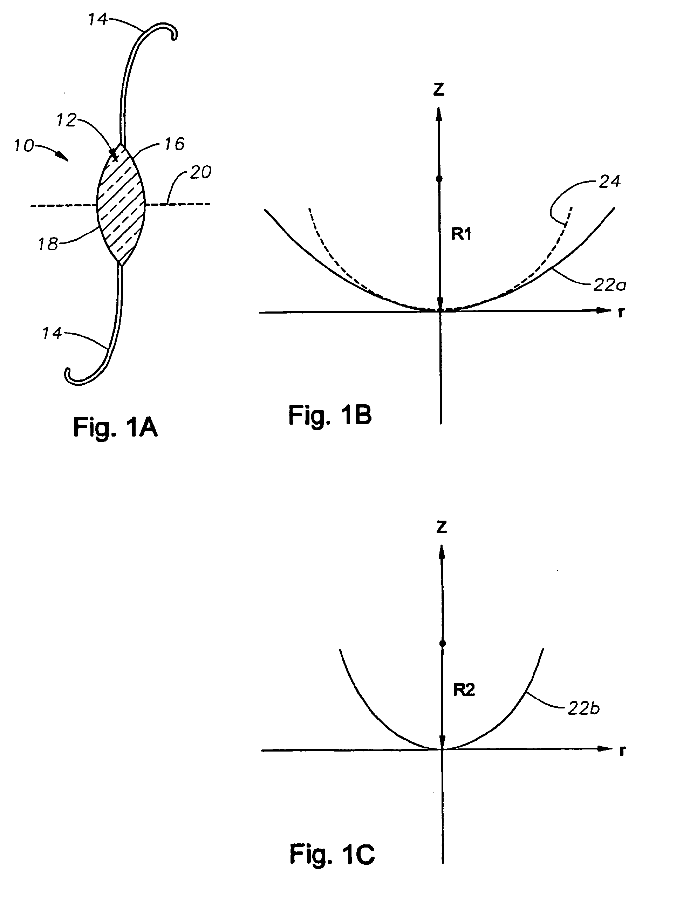

FIGURE 1A schematically depicts a monofocal intraocular lens 10 according to one embodiment of the invention having an optic 12 preferably formed of a soft biocompatible material, such as soft acrylic polymer, silicone, or hydrogel. The exemplary lens 10 further includes radially extending fixation members or haptics 14 for its placement in a patient's eye. The fixation members 14 can be made of suitable polymeric materials, such as polypropylene, polymethyl methacrylate and the like as known to those having ordinary skill in the art. In some embodiments, the optic and the fixation members are formed from the same material as a single -piece lens. The optic 12 includes an anterior refractive surface 16 and a posterior refractive surface 18 that are shaped so as to cooperatively provide the lens with a nominal optical power in a range of zero to about 40 Diopters and, more preferably, in a range of about 18 to about 26 Diopters. In this exemplary embodiment, the refractive surfaces 16 and 18 are generally symmetric about an optical axis 20 of the lens, although in other embodiments either surface can be asymmetric about this axis. Further, although the refractive surfaces 16 and 18 are depicted as being generally convex, either surface can have a generally concave shape. Alternatively, the surfaces 16 and 18 can be selected to generate a plano-convex or a plano-concave lens. Hence, a lens according to the teachings of the invention can have a positive or a negative nominal power. In some embodiments, the lens can have a negative power, e.g., in a range of about -20 D to about -10 D, or -15 D to about -10 D. Such lenses can be employed in phakic patients. More generally, a lens of the invention can have a power in a range of about -20 D to about +10 D.

FIGURE 1B schematically illustrates a base profile 22a of the anterior refractive surface 16 as a function of radial distance (r) relative to an intersection of optical axis 20 with the anterior surface 16 (for purposes of illustration the curvature is greatly exaggerated). In this embodiment, the base profile 22a is aspherical with a selected degree of deviation from a putative spherical profile 24 having a radius of curvature R$_1$ that substantially coincides with the aspherical profile at small radial distances. Although in this exemplary embodiment the aspherical anterior surface 16 is flatter than the putative spherical profile, in other embodiments it can be steeper. The posterior surface 18 exhibits a spherical profile 22b with a radius of curvature R$_2$, as shown schematically in FIGURE 1C. The radii R$_1$ and R$_2$ are generally chosen to provide the lens with a desired optical power and a desired shape factor. In other embodiments, the posterior surface can also exhibit an aspheric profile, while in others the anterior surface can be spherical and the posterior surface aspherical. In other words, a desired degree of asphericity can be achieved by imparting an aspherical profile to only one of the refractive surfaces, or by dividing the total aspheric deviation between the two surfaces.

Referring again to FIGUREs 1A and 1B, in many embodiments, the aspheric profile of the anterior surface 16 is selected to control the aberrations of a patient's eye in which the IOL 10 is implanted so as to enhance the patient's image contrast relative to that provided by a substantially identical lens in which the anterior surface has the putative spherical profile 24, rather than the aspherical profile 22a, while providing the patient with a depth of field greater than about 0.75 D. More specifically, in many embodiments, the aspheric profile controls the aberrations of an eye in which the IOL 10 is implanted such that the combined lens and cornea, or the combined lens, the cornea and the natural lens, would exhibit a peak modulation transfer function (MTF) contrast of at least about 0.25 and a depth of field of at least about 0.75 Diopters for pupil diameters in a range of about 4.5 millimeters to about 5 millimeters when measured or calculated with monochromatic light at a wavelength of about 550 nanometers and at a spatial frequency of about 50 line pairs per millimeter. For example, the patient having the IOL can experience a peak MTF contrast at the retina in a range of about 0.25 to about 0.4 while having a depth of focus in a range of about 0.75 to about 1.5 D. In this manner, the image contrast is enhanced while maintaining a useful depth of field.

As known to those having ordinary skill in the art, a quantitative measure of image contrast provided by a lens can be obtained by calculating and/or measuring a modulation transfer function (MTF) associated with that lens. In general, a contrast or modulation associated with an optical signal, e.g., a two-dimensional pattern of light intensity distribution emanated from or reflected by an object to be imaged or associated with the image of such an object, can be defined in accordance with the following relation:

$$\frac{I_{max} - I_{min}}{I_{max} + I_{min}}$$

wherein $I_{max}$ and $I_{min}$ indicate, respectively, a maximum or a minimum intensity associated with the signal. Such a contrast can be calculated or measured for each spatial frequency present in the optical signal. An MTF of an imaging optical system, such as the combined IOL and the cornea, can then be defined as a ratio of a contrast associated with an image of an object formed by the optical system relative to a contrast associated with the object. As is known, the MTF associated with an optical

system is not only dependent on the spatial frequencies of the intensity distribution of the light illuminating the system, but it can also be affected by other factors, such as the size of an illumination aperture as well as the wavelength of the illuminating light.

Although in many embodiments an IOL according to the invention is utilized to enhance a patient's image contrast, in some embodiments, it can be employed to primarily enhance a patient's depth of field with a potential moderate decrease in the image contrast. For example, a patient whose cornea exhibits a highly aspherical flattening can benefit from an aspherical IOL according to one embodiment of the invention that can partially compensate for the severe flattening to enhance the patient's depth of filed albeit with a potential small decrease in the image contrast.

In some embodiments, the aspherical profile of the anterior surface 16 of the IOL 10 as a function of radial distance (R) from the optical axis 20, or that of the posterior surface or both in other embodiments, can be characterized by the following relation:

$$z = \frac{CR^2}{1 + \sqrt{1-(1+Q)C^2R^2}} + AR^4 + BR^6 + \text{ higher order terms},$$

wherein

$z$ denotes a sag of the surface parallel to an axis (z), e.g., the optical axis, perpendicular to the surface,

$C$ denotes a curvature at the vertex of the surface,

$Q$ denotes a conic coefficient,

$R$ denotes a radial position on the surface,

$A$ denotes a fourth order deformation coefficient, and

$B$ denotes a sixth order deformation coefficient.

In many embodiments, the conic constant $Q$ alone can be adjusted to obtain a desired deviation from sphericity with the higher order aspherical constants $A$ and $B$, and others set to zero. In other embodiments, one or both of the higher order constants $A$ and $B$, in addition to, or instead of, the conic constant $Q$, can be adjusted to provide a selected aspherical profile for one or both refractive surfaces of an IOL. The higher order aspherical constants can be particularly useful for tailoring the profile of the peripheral portions of the lens surface, i.e., portions far from the optical axis.

The choice of the aspherical constants can depend, for example, on the aberrations of the eye in which the IOL is implanted, the material from which the IOL is fabricated, and the optical power provided by the IOL. In general, these constants are selected such that the IOL can provide a balance between a patient's image contrast and depth of field, e.g., enhancing the image contrast while substantially preserving the depth of field. For example, in some embodiments in which the IOL is fabricated from an acrylic polymeric material for implantation in an eye exhibiting a corneal asphericity characterized by a corneal conic constant in the range of zero (associated with severe spherical aberration) to about - 0.5 (associated with a high level of aspherical flattening) the conic constant $Q$ of the lens in the above relation can be in a range of about 0 to about - 50 , while the deformation coefficients $A$ and $B$ can be, respectively, in a range of about - 1 x $10^{-3}$ to about 1x $10^{-3}$ and in a range of about -1 x $10^{-4}$ to about 1 x $10^{-4}$.

As noted above, the choice of the degree of sphericity of one or both surfaces of the IOL can depend, at least in part, on the lens's shape factor $(X)$. For examples, in some embodiments in which the IOL exhibits a shape factor in a range of about 0 to about +1, the conic constant can be in range of about -50 to about 0.

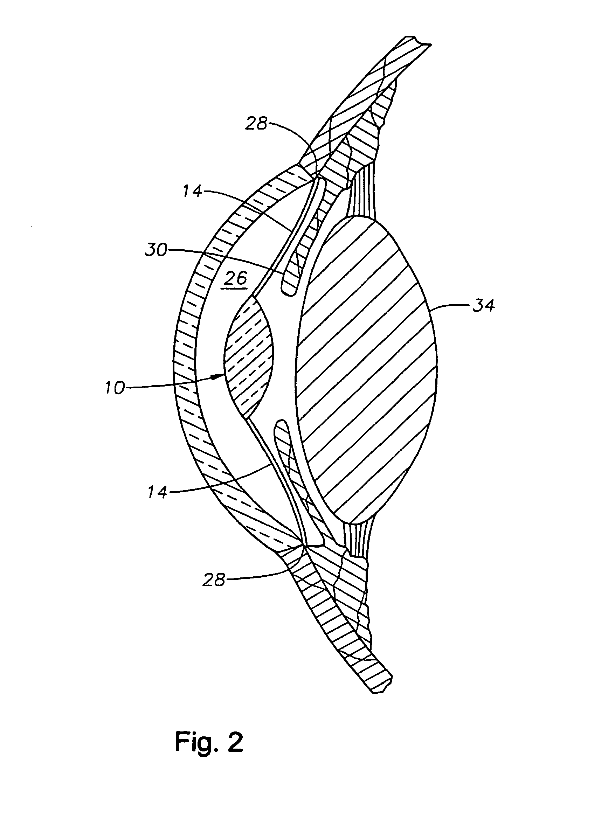

An IOL according to the teachings of the invention can find a variety of different applications. By way of example, in phakic patients, the IOL 10 can be implanted in a patient's eye while retaining the eye's natural lens by inserting the optic 12 in the eye's anterior chamber 26 with the distal ends of the fixation members 14 in contact with an angle 28 of the iris 30, as shown in FIGURE 2. The IOL can provide an optical power for correcting a refractive defect of the eye. The aspherical profile of the anterior surface 16 of the IOL can control the overall aberrations of the natural eye, for example, the combined aberrations of the cornea 32 and the natural lens 34, so as to enhance the image contrast on the retina, especially for large pupil sizes, while maintaining a desired depth of field, as discussed above.

In another application, in pseudophakic patients, the IOL 10 can be implanted in a patient's eye after removal of the patient's natural lens during cataract surgery. The aspherical profile of the IOL 10 can control the aberrations exhibited by the cornea so as to enhance the image contrast while substantially preserving the depth of field. In some cases, the cornea is substantially spherical, characterized, for example, by a vanishing conic constant, while in other cases the cornea itself can show a certain degree of asphericity. The aspherical profile of the IOL 10 can be adjusted accordingly to provide the desired degree of image contrast, or depth of field, enhancement. For example, in some cases, the aspherical profile of IOL can be characterized by curve that is flatter than that of a putative spherical profile, while in other cases it is steeper.

A variety of techniques can be employed to determine the requisite degree of asphericity for the IOL 10. For example, in one approach, aberrations exhibited by a patient's eye, or by a group of patients, are measured preoperatively by employing known topographical methods and systems. For phakic eyes, the measured aberrations can correspond primarily to the combined aberrations of the natural lens and the cornea while for the pseudophakic patients, they can correspond to those of the cornea. In one such method, wavefront aberrations of the eye can be measured at a selected measurement plane, e.g., the entrance pupil of the patient's eye, and at a selected wavelength, e.g., at a wavelength of about 830 nm. Further details regarding measurements of aberrations of the eye can be found in U.S. Patent No. 6,786,603 and U.S. Patent Application No. 2002/0105617.

Wavefront measurements can be employed to determine a requisite level of asphericity of the IOL needed for controlling the aberrations of the eye. For example, an aspherical profile of the IOL can be designed to reduce spherical aberrations of the cornea inferred from the wavefront measurements. One or more aspherical parameters of the IOL can be obtained theoretically or experimentally, or both. For example, a ray tracing program, such as OSLO, marketed by Lambda Research Corporation of Littleton, Massachusetts, U.S.A, can be employed to model the eye and its aberrations inferred from the wavefront measurements as well as an IOL having one or more aspherical surfaces. The asphericity of the IOL can then be adjusted, e.g., via adjusting the conic constant and possibly the higher order deformation constants, to obtain a desired MTF and depth of field. In some cases, average aberrations exhibited by the eyes of a selected group of patients are considered for designing an IOL suitable for controlling on average such aberrations.

The aspherical parameters of an IOL according to the teachings of the invention can also be determined experimentally. For example, an IOL can be inserted in a model eye exhibiting aberrations corresponding to those inferred from the wavefront measurements, e.g., a corneal aberration characterized by a conic constant. Subsequently, modulation transfer functions obtained by a combined system of the model eye and intraocular lenses having different aspherical profiles are measured to select a suitable aspherical profile.

As noted above, in some cases, the aberrations of a population of patients are measured to design a lens suitable for controlling average aberrations exhibited by patients. For example, in some cases, two or more types of intraocular lenses, each type designed to control average aberrations exhibited by the eyes of select group of patients, can be provided.

To demonstrate the efficacy of intraocular lenses according to the teachings of the invention to provide a more useful balance between the image contrast and the depth of field, theoretical ray tracing calculations were performed to determine modulation transfer functions exhibited by a combined system of an IOL according to the teachings of the invention having an aspherical profile and an eye modeled to have a selected corneal aberration in a range typically exhibited by patients in the general population. More specifically, for each theoretically modeled lens, the modulation transfer function at 50 line pairs per millimeter (lp/mm) and at a wavelength of about 550 nm, as well as a depth of focus, were calculated for the combined lens and cornea, as discussed below. In addition, corresponding control calculations of MTF and depth of focus were performed for a substantially identical IOL having a spherical profile, i.e., an IOL exhibiting a vanishing conic constant. The depth of field was determined as the amount of defocus about a nominal focus corresponding to the peak MTF at which the MTF value drops to about 0.05.

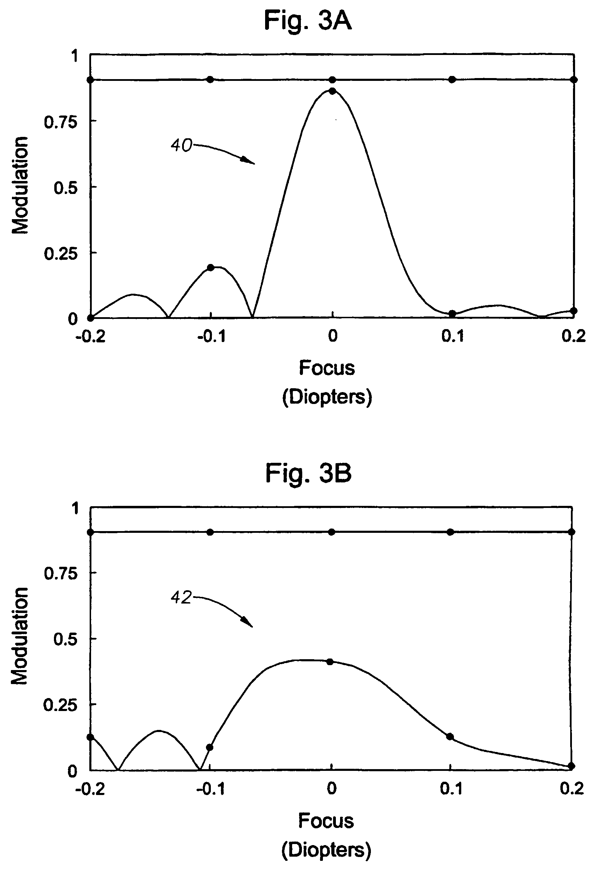

As another example, FIGURE 3A presents a through-focus MTF plot 40 for a cornea having a conic constant of - 0.5 - a cornea exhibiting a high level of aspheric flattening - with an IOL having a vanishing conic constant (herein referred to as condition A), while FIGURE 3B presents a respective MTF plot 42 for the same cornea but with an IOL according to the teachings of the invention having an aspherical anterior surface with a conic constant of 2.8 (herein referred to as condition B). A comparison of the plots 40 and 42 shows that although the cornea with the aspherical lens exhibits a lower peak MTF contrast (an MTF of 0.41 for the aspherical case relative to 0.86 for the spherical case), it exhibits, however, a much improved depth of field (a depth of field of 0.8 for the aspherical case compared with 0.50 for the spherical case). Hence, in some cases the asphericity of the IOL is selected to reduce an asphericity exhibited by a cornea so as to enhance the depth of field at the expense of a slight decrease in the image contrast.

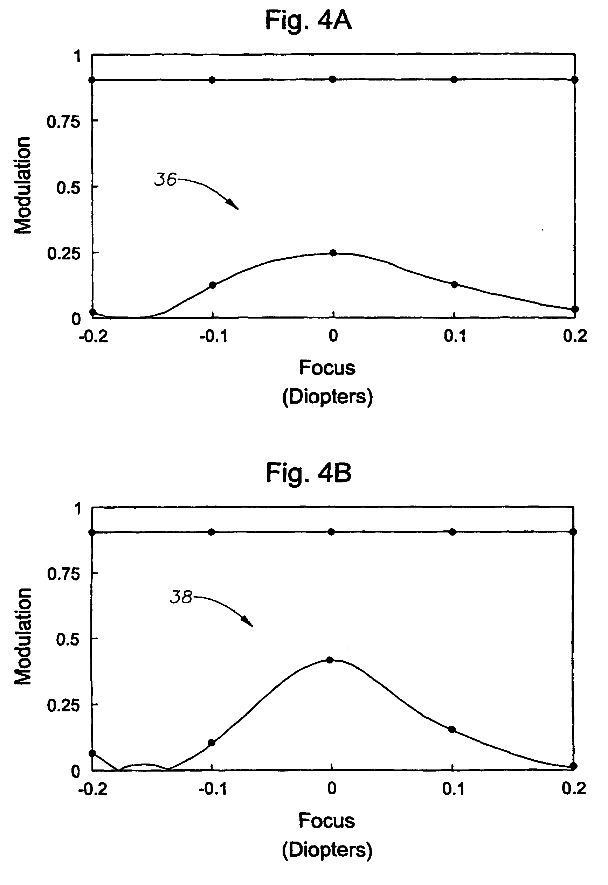

FIGURE 4A presents a calculated control through-focus modulation transfer function plot 36 for a spherical cornea - a cornea exhibiting severe spherical aberration - combined with an IOL having a spherical profile (i.e., a conic constant of zero), herein referred to as condition E, while FIGURE 4B presents a respective through-focus MTF plot 38 for the same cornea combined with an IOL according to the teachings of the invention having an anterior aspherical surface with a conic constant of - 6, herein referred to as condition D. A comparison of plots 36 and 38 shows that the use of the aspherical lens results in an increase of the peak MTF contrast from about 0.24 to about 0.4 (a 67 % increase), while substantially preserving the depth of focus (a depth of focus of 1.14 Diopters at 0.05 MTF for the spherical lens compared to a corresponding depth of focus of 1.02 Diopters for the aspherical lens).

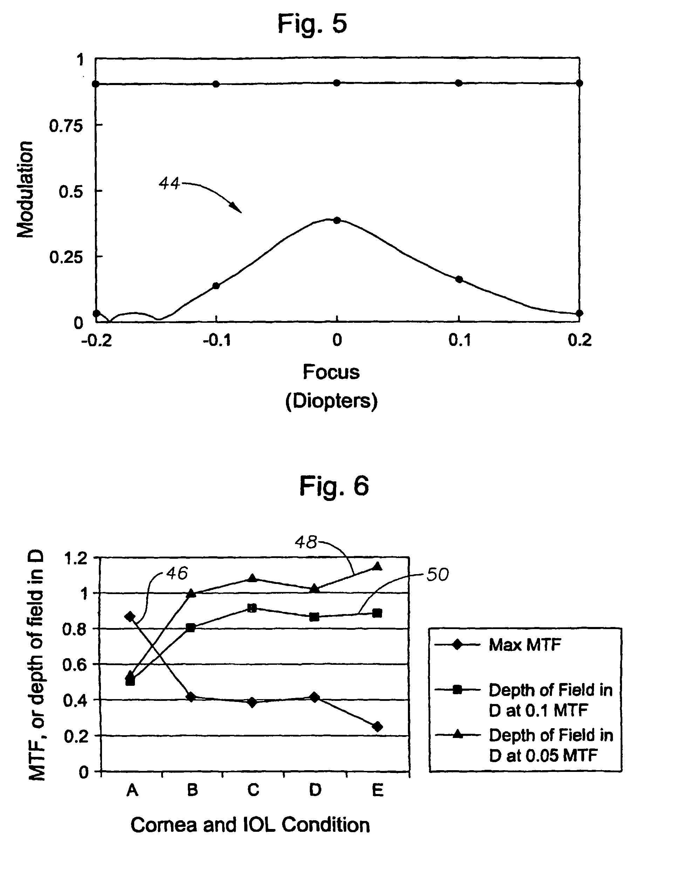

An average patient may have a cornea with an asphericity characterized by a conic constant of -0.26. Although no calculation was performed for such a cornea with an aspherical IOL, FIGURE 5 shows a through-focus MTF plot 44 of such a cornea with a spherical IOL (herein referred to as condition C), indicating a peak MTF comparable with those obtained for the above conditions B and D, namely, for a spherical cornea with an aspherical lens having a negative conic constant of -6 and a cornea exhibiting severe aspherical flattening with an aspherical lens having a positive conic constant of 2.8.

To summarize the data discussed above for the exemplary conditions A-E, FIGURE 6 presents three graphs 46, 48 and 50 illustrating, respectively, peak MTF, and defocus in Diopter (D) at which the MTF has a value of 0.05 and 0.1 (herein also referred to as depth of focus at 0.05 or 0.1 MTF). For example, these graphs show that the peak MTF increases by employing an IOL according to the teachings of the invention having an aspheric profile for an eye with a spherical cornea while substantially preserving the depth of field.

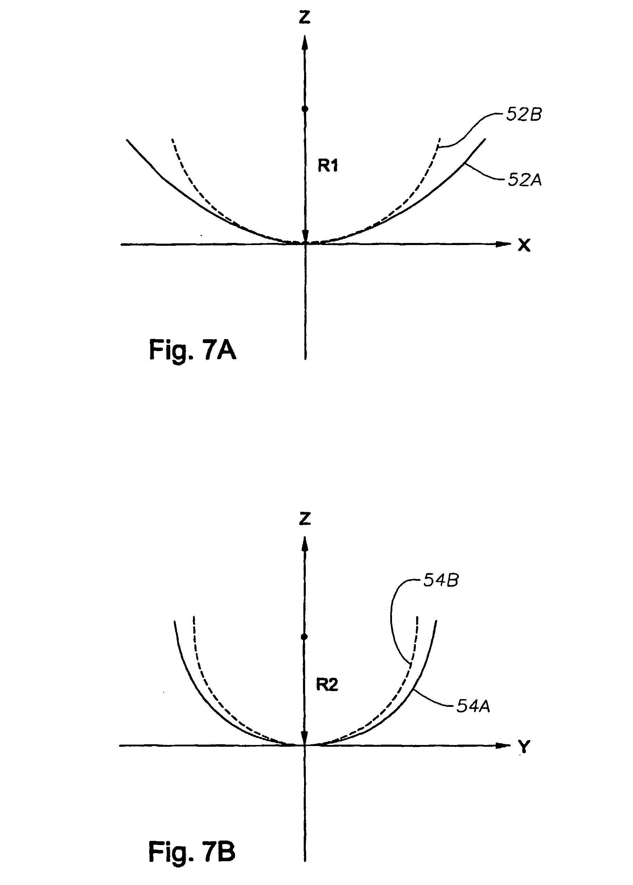

In another embodiment, an intraocular lens (IOL) of the invention can have one or two toric refractive surfaces that exhibit two different optical powers along two orthogonal surface directions. Such toric IOLs can be employed, for example, to correct astigmatism. In some embodiments, one surface is toric and the other non-toric. A selected degree of asphericity can be imparted to the toric surface, to the non-toric surface or to both. Alternatively, both lens surfaces can be toric with at least one exhibiting asphericity. For example, at least one of the toric surfaces can exhibit an asphericity along one or both of the two surface orthogonal directions, each associated with an optical power different than the power along the other direction, such that a combination of the lens and the eye in which the lens is implanted provides not only a useful image contrast, but also a depth of field within an acceptable range, such as those discussed above in connection with the other embodiments. For example, with reference to FIGURE 7A, the toric surface exhibiting a selected asphericity in one of the two directions (herein identified with the x coordinate) can be characterized by an aspherical profile 52A having a central curvature $R_1$ at its vertex (i.e., intersection of an optical axis of the lens with the surface) and a selected deviation from a putative spherical profile 52B that substantially coincides with the aspherical profile at small radial distances. As shown in FIGURE 7B, along the other direction (herein identified with the y coordinate), a profile 54A of the toric surface can be characterized by a central curvature $R_2$, that is different than $R_1$, and a selected deviation from a putative spherical profile 54B that substantially coincides with the aspherical profile at small radial distances.

Those having ordinary skill in the art will appreciate that various modifications can be made to the above embodiments without departing from the scope of the invention.

**Claims**

1. An intraocular lens (IOL) (10), comprising:

   a refractive optic (12) having an anterior surface (16) and a posterior surface (18),
   at least one of said anterior or posterior surfaces having an aspherical profile adapted for controlling the aberrations of a model eye in which the IOL is implanted and to provide the model eye with an image contrast,
   **characterized in that** a combined lens and model cornea exhibit a peak calculated modulation transfer function (MTF) contrast of at least about 0.25 and a depth of field of at least about 0.75 diopters, for pupil diameters in a range of 4.5 mm to 5 mm for monochromatic light at a wavelength of about 550 nm,
   wherein said anterior surface (16) is **characterized by** said aspheric profile exhibiting a selected deviation from a putative spherical profile having a radius of curvature $R_1$,
   and said posterior base surface (18) having a base profile with a radius of curvature of $R_2$, wherein $R_2$ is larger than $R_1$.

2. The IOL of claim 1, wherein said combined lens and model cornea exhibit a modulation transfer function (MTF) at the retina greater than about 0.3 for 50 line pairs per mm and a wavelength of about 550 nm.

3. The IOL of claim 1, wherein said combined lens and model cornea exhibit a modulation transfer function (MTF) greater than about 0.35 for 50 line pairs per mm and a wavelength of about 550 nm.

4. The IOL of claim 1, wherein said combined lens and model cornea exhibit a modulation transfer function (MTF) in a range of about 0.25 to about 0.4 at a spatial frequency of about 50 lp/mm, a wavelength of about 550 nm and a pupil size of about 4.5 mm.

5. The IOL of claim 1, wherein said posterior surface (18) is **characterized by** a spherical profile having a radius of curvature of $R_2$, wherein $R_2$ is larger than $R_1$.

6. The IOL of claim 5, wherein said lens exhibits a shape factor K defined as:

$$X = \frac{R_2 + R_1}{R_2 - R_1},$$

   wherein K is in a range of about 0 to about +1.

7. The IOL of claim 1, wherein said posterior surface (18) is **characterized by** an aspheric base profile exhibiting a selected deviation from a putative spherical profile having a radius of curvature of $R_2$, wherein $R_2$ is larger than $R_1$.

8. The IOL of any of claims 1 to 7, wherein said aspherical profile is adapted for controlling a spherical aberration of the model cornea.

9. The IOL of any of claim 1 to 8, wherein said aspherical surface comprises an anterior refractive surface (16) of said lens.

10. The IOL of any of claims 1 to 8, wherein said aspherical surface comprises a posterior refractive surface (18) of said lens.

11. The IOL of any of claims 1 to 10, having a diopter optical power is in a range of about 0 to about 40.

12. The IOL of any of claims 1 to 11, wherein said lens is adapted to provide the patient with an MTF in a range of 0.25 to 0.4.

13. The IOL of any of claims 1 to 12, wherein said lens is adapted to provide the model eye with a depth of field of in a range of 0.75 to 1.5 diopters.

14. The IOL of any of claims 1 to 13, wherein said aspherical profile is adapted to control aberrations exhibited by the model cornea.

15. The IOL of any of claims 1 to 13, wherein said aspherical profile is adapted to control aberrations exhibited by the combined model cornea and a natural lens.

16. The IOL of any of claims 1 to 13, wherein said aspherical profile is adapted to control average aberrations exhibited by the eyes of a selected patient group.

17. The IOL of claim 1, comprising an optic comprising at least one refractive surface having a base **characterized by** a profile described by the following relation:

$$z = \frac{CR^2}{1+\sqrt{1-(1+Q)C^2R^2}} + AR^4 + BR^6 \,,$$

wherein $z$ denotes a sag of the surface parallel to an axis (z) perpendicular to the surface,
$C$ denotes a curvature at the vertex of the surface,
$Q$ denotes a conic coefficient,
$R$ denotes a radial position on the surface,
$A$ denotes a fourth order deformation coefficient, and
$B$ denotes a sixth order deformation coefficient,
wherein $Q$ is in a range of about 0 to about 100, $A$ is in a range of about $-1x\ 10^{-3}$ to about $1x\ 10^{-3}$, and $B$ is in a range of about $-1x\ 10^{-4}$ to about $1x\ 10^{-4}$.

18. The IOL of any of claims 1 to 17, wherein said optic is formed of a biocompatible soft material, including a soft acrylic material, a hydrogel, or silicone.

19. The IOL of claim 18, wherein said optic exhibits an aspherical conic constant in a range of about 0 to about -50.

20. The IOL of claim 1, comprising an optic (12) having an anterior refractive surface (16) and a posterior refractive surface (18), at least one of said surfaces having a generally toric shape exhibiting different optical power values along two orthogonal surface directions and having an asphericity along at least one of said orthogonal directions for controlling aberrations of a model eye in which the IOL is implanted such that the combination of the lens and the model eye exhibits a modulation transfer function of at least about 0.25 and a depth of field of at least about 0.75 D for a pupil size of about 4.5 mm and a monochromatic wavelength of about 550 nm as calculated in the model eye.

21. A method of designing an intraocular lens (10) having an anterior and a posterior refractive surface (16,18), comprising:

deriving a model average of aberrations of a model eye based on wavefront measurements of aberrations exhibited by the eyes of a selected patient population, and
adjusting asphericity of at least one of said refractive surfaces for controlling said average aberrations such that a model eye in which the lens is implanted would exhibit an image contrast **characterized by** a peak modulation transfer function (MTF) contrast of at least about 0.25 and a depth of field of at least about 0.75 D for pupil diameters in a range of 4.5 mm to 5 mm for monochromatic light at a wavelength of about 550 nm.

**Patentansprüche**

1. Intraokularlinse (IOL) (10), aufweisend:

   eine refraktive Optik (12), die eine vordere Fläche (16) und eine hintere Fläche (18) hat,
   wobei zumindest eine der vorderen oder hinteren Fläche ein asphärisches Profil hat, das ausgelegt ist, die Abbildungsfehler eines Modellauges, in das die IOL implantiert ist,
   zu regulieren und einen Bildkontrast für das Modellauge bereitzustellen,
   **dadurch gekennzeichnet, dass** eine Kombination einer Linse und einer Modellhornhaut einen auf Spitzenwerte berechneten Modulationsübertragungsfunktions(MÜF)-Kontrast von zumindest etwa 0,25 und eine Feldtiefe von zumindest etwa 0,75 Dioptrien aufweist, für Pupillendurchmesser in einem Bereich von 4,5 mm bis 5 mm und für monochromatisches Licht mit einer Wellenlänge von etwa 550 nm,
   wobei die vordere Fläche (16) durch das asphärische Profil **gekennzeichnet** ist, das eine gewählte Abweichung von einem vermeintlich sphärischen Profil mit einem Krümmungsradius von $R_1$ aufweist,
   und die hintere Basisfläche (18) ein Basisprofil mit einem Krümmungsradius von $R_2$ hat, wobei $R_2$ größer als $R_1$ ist.

2. IOL nach Anspruch 1, wobei die Kombination aus Linse und Modellhornhaut an der Netzhaut eine Modulationsübertragungsfunktion (MÜF) aufweist, die größer als etwa 0,3 für 50 Linienpaare pro mm und eine Wellenlänge von etwa 550 nm ist.

3. IOL nach Anspruch 1, wobei die Kombination aus Linse und Modellhornhaut eine Modulationsübertragungsfunktion (MÜF) aufweist, die größer als etwa 0,35 für 50 Linienpaare pro mm und eine Wellenlänge von etwa 550 nm ist.

4. IOL nach Anspruch 1, wobei die Kombination aus Linse und Modellhornhaut eine Modulationsübertragungsfunktion (MÜF) in einem Bereich von etwa 0,25 bis etwa 0,4 auf einer Ortsfrequenz von etwa 50 LP/mm, einer Wellenlänge von etwa 550 nm und bei einer Pupillengröße von etwa 4,5 mm aufweist.

5. IOL nach Anspruch 1, wobei die hintere Fläche (18) durch ein sphärisches Profil **gekennzeichnet** ist, das einen Krümmungsradius von $R_2$ hat, wobei $R_2$ größer als $R_1$ ist.

6. IOL nach Anspruch 5, wobei die Linse einen Formfaktor K aufweist, der definiert ist als:

$$X = \frac{R_2 + R_1}{R_2 - R_1},$$

   wobei K in einem Bereich von etwa 0 bis etwa +1 liegt.

7. IOL nach Anspruch 1, wobei die hintere Fläche (18) durch ein asphärisches Basisprofil **gekennzeichnet** ist, das eine gewählte Abweichung von einem vermeintlich sphärischen Profil mit einem Krümmungsradius von $R_2$ aufweist, wobei $R_2$ größer als $R_1$ ist.

8. IOL nach einem der Ansprüche 1 bis 7, wobei das asphärische Profil ausgelegt ist, einen sphärischen Abbildungsfehler der Modellhornhaut zu regulieren.

9. IOL nach einem der Ansprüche 1 bis 8, wobei die asphärische Fläche eine vordere refraktive Fläche (16) der Linse aufweist.

10. IOL nach einem der Ansprüche 1 bis 8, wobei die asphärische Fläche eine hintere refraktive Fläche (18) der Linse aufweist.

11. IOL nach einem der Ansprüche 1 bis 10, die eine Brechkraft in einem Bereich von etwa 0 bis etwa 40 Dioptrien hat.

12. IOL nach einem der Ansprüche 1 bis 11, wobei die Linse ausgelegt ist, dem Patienten eine MÜF in einem Bereich von 0,25 bis 0,4 bereitzustellen.

**13.** IOL nach einem der Ansprüche 1 bis 12, wobei die Linse ausgelegt ist, dem Modellauge eine Feldtiefe in einem Bereich von 0,75 bis 1,5 Dioptrien bereitzustellen.

**14.** IOL nach einem der Ansprüche 1 bis 13, wobei das asphärische Profil ausgelegt ist, Abbildungsfehler zu regulieren, die bei der Modellhornhaut auftreten.

**15.** IOL nach einem der Ansprüche 1 bis 13, wobei das asphärische Profil ausgelegt ist, Abbildungsfehler zu regulieren, die bei der Kombination der Modellhornhaut und einer natürlichen Linse auftreten.

**16.** IOL nach einem der Ansprüche 1 bis 13, wobei das asphärische Profil ausgelegt ist, durchschnittliche Abbildungsfehler zu regulieren, die an den Augen einer ausgewählten Gruppe von Patienten auftreten.

**17.** IOL nach Anspruch 1, umfassend eine Optik, die zumindest eine refraktive Fläche mit einer Basis aufweist, die durch ein von der folgenden Relation beschriebenes Profil **gekennzeichnet** ist:

$$z = \frac{CR^2}{1 + \sqrt{1 - (1+Q)C^2R^2}} + AR^4 + BR^6,$$

wobei z eine Durchbiegung der Fläche bezeichnet, die parallel zu einer Achse (z) ist, die rechtwinklig auf die Fläche steht,
C eine Krümmung am Scheitel der Fläche bezeichnet,
Q einen konischen Koeffizienten bezeichnet,
R eine radiale Position auf der Fläche bezeichnet,
A einen Koeffizienten für eine Deformation vierter Ordnung bezeichnet, und
B einen Koeffizienten für eine Deformation sechster Ordnung bezeichnet,
wobei Q in einem Bereich von etwa 0 bis etwa 100 liegt, A in einem Bereich von etwa $-1 \times 10^{-3}$ bis etwa $1 \times 10^{-3}$ liegt und B in einem Bereich von etwa $-1 \times 10^{-4}$ bis etwa $1 \times 10^{-4}$ liegt.

**18.** IOL nach einem der Ansprüche 1 bis 17, wobei die Optik aus einem biokompatiblen weichen Material gebildet ist, einschließlich eines weichen Acrylmaterials, eines Hydrogels oder Silikon.

**19.** IOL nach Anspruch 18, wobei die Optik eine asphärische konische Konstante in einem Bereich von etwa 0 bis etwa -50 aufweist.

**20.** IOL nach Anspruch 1, umfassend eine Optik (12), die eine vordere refraktive Fläche (16) und eine hintere refraktive Fläche (18) hat, wobei zumindest eine der Flächen eine im Allgemeinen torische Form hat, die entlang zweier orthogonaler Flächenrichtungen verschiedene Brechkraftwerte aufweist und entlang zumindest einer der orthogonalen Richtungen eine Asphärizität zum Regulieren von Abbildungsfehlern eines Modellauges hat, in das die IOL implantiert ist, sodass die Kombination der Linse und des Modellauges eine Modulationsübertragungsfunktion von zumindest etwa 0,25 und eine Feldtiefe von zumindest etwa 0,75 D für eine Pupillengröße von etwa 4,5 mm und eine monochromatische Wellenlänge von etwa 550 nm aufweist, wie diese im Modellauge berechnet sind.

**21.** Verfahren zum Gestalten einer Intraokularlinse (10), die eine vordere und eine hintere refraktive Fläche (16, 18) hat, umfassend:

Ableiten eines Modelldurchschnitts von Abbildungsfehlern eines Modellauges, basierend auf Messungen der Wellenfronten von Abbildungsfehlern, die an den Augen einer ausgewählten Patientenpopulation auftreten, und Anpassen einer Asphärizität zumindest einer der refraktiven Flächen zum Regulieren der durchschnittlichen Abbildungsfehler, sodass ein Modellauge, in das die Linse implantiert ist, einen Bildkontrast aufweisen würde, der durch einen Spitzenwert eines Modulationsübertragungsfunktions(MÜF)-Kontrastes von zumindest etwa 0,25 und eine Feldtiefe von zumindest etwa 0,75 D **gekennzeichnet** ist, für Pupillendurchmessern in einem Bereich von 4,5 mm bis 5 mm und monochromatisches Licht mit einer Wellenlänge von etwa 550 nm.

**Revendications**

1. Lentille intraoculaire (LIO) (10) comprenant :

   une lentille optique réfractive (12) ayant une surface antérieure (16) et une surface postérieure (18),
   au moins une des faces antérieure ou postérieure ayant un profil asphérique adapté pour contrôler les aberrations d'un oeil modèle dans lequel la LIO est implantée, et pour apporter du contraste d'image à l'oeil modèle, **caractérisée en ce qu'**une lentille et une cornée modèle combinées présentent un contraste de fonction de transfert de modulation (FTM) calculé maximum d'au moins environ 0,25 et une profondeur de champ d'au moins environ 0,75 dioptrie, pour des diamètres de pupille s'étendant de 4,5 mm à 5 mm, pour une lumière monochromatique d'une longueur d'onde d'environ 550 nm,
   dans laquelle ladite surface antérieure (16) est **caractérisée par** ledit profil asphérique présentant une déviation sélectionnée par rapport à un profil sphérique putatif ayant un rayon de courbure $R_1$,
   et ladite surface de base postérieure (18) ayant un profil de base avec un rayon de courbure $R_2$, où $R_2$ est plus grand que $R_1$.

2. Lentille LIO selon la revendication 1, dans laquelle lesdites lentille et cornée modèle combinées présentent une fonction de transfert de modulation (FTM) au niveau de la rétine supérieure à environ 0,3 pour 50 paires de lignes par mm et à une longueur d'onde d'environ 550 nm.

3. Lentille LIO selon la revendication 1, dans laquelle lesdites lentille et cornée modèle combinées présentent une fonction de transfert de modulation (FTM) supérieure à environ 0,35 pour 50 paires de lignes par mm et à une longueur d'onde d'environ 550 nm.

4. Lentille LIO selon la revendication 1, dans laquelle lesdites lentille et cornée modèle combinées présentent une fonction de transfert de modulation (FTM) s'étendant d'environ 0,25 à environ 0,4 à une fréquence spatiale d'environ 50 lp/mm, à une longueur d'onde d'environ 550 nm et un diamètre de pupille d'environ 4,5 mm.

5. Lentille LIO selon la revendication 1, dans laquelle ladite surface postérieure (18) est **caractérisée par** un profil sphérique ayant un rayon de courbure $R_2$, où $R_2$ est plus grand que $R_1$.

6. Lentille LIO selon la revendication 5, dans laquelle ladite lentille présente un facteur de forme K défini comme suit :

$$K = \frac{R_2 + R_1}{R_2 - R_1}$$

où K est compris entre environ 0 et environ +1.

7. Lentille LIO selon la revendication 1, dans laquelle ladite surface postérieure (18) est **caractérisée par** un profil de base asphérique présentant une déviation sélectionnée par rapport à un profil sphérique putatif ayant un rayon de courbure $R_2$, où $R_2$ est plus grand que $R_1$.

8. Lentille LIO selon l'une quelconque des revendications 1 à 7, dans laquelle ledit profil asphérique est adapté pour contrôler une aberration sphérique de la cornée modèle.

9. Lentille LIO selon l'une quelconque des revendications 1 à 8, dans laquelle ladite surface asphérique comprend une surface réfractive antérieure (16) de ladite lentille.

10. Lentille LIO selon l'une quelconque des revendications 1 à 8, dans laquelle ladite surface asphérique comprend une surface réfractive postérieure (18) de ladite lentille.

11. Lentille LIO selon l'une quelconque des revendications 1 à 10, ayant une puissance optique dioptrique s'étendant d'environ 0 à environ 40.

12. Lentille LIO selon l'une quelconque des revendications 1 à 11, dans laquelle ladite lentille est adaptée pour procurer au patient une FTM s'étendant de 0,25 à 0,4.

**13.** Lentille LIO selon l'une quelconque des revendications 1 à 12, dans laquelle ladite lentille est adaptée pour apporter à l'oeil modèle une profondeur de champ s'étendant de 0,75 à 1,5 dioptrie.

**14.** Lentille LIO selon l'une quelconque des revendications 1 à 13, dans laquelle ledit profil asphérique est adapté pour contrôler les aberrations présentées par la cornée modèle.

**15.** Lentille LIO selon l'une quelconque des revendications 1 à 13, dans laquelle ledit profil asphérique est adapté pour contrôler les aberrations présentées par la cornée modèle et un cristallin naturel combinés.

**16.** Lentille LIO selon l'une quelconque des revendications 1 à 13, dans laquelle ledit profil asphérique est adapté pour contrôler les aberrations présentées par les yeux d'un groupe de patients sélectionné.

**17.** Lentille LIO selon la revendication 1, comprenant une lentille optique comprenant au moins une surface réfractive ayant une base **caractérisée par** un profil décrit par l'équation suivante :

$$z = \frac{CR^2}{1 + \sqrt{1 - (1 + Q)C^2R^2}} + AR^4 + BR^6$$

où z désigne un gauchissement de la surface parallèle à un axe (z) perpendiculaire à la surface,
C désigne une courbure au niveau du vertex de la surface,
Q désigne un coefficient conique,
R désigne une position radiale sur la surface,
A désigne un coefficient de déformation de quatrième ordre, et
B désigne un coefficient de déformation de sixième ordre,
où Q se situe dans un intervalle s'étendant d'environ 0 à environ 100, A dans un intervalle s'étendant d'environ $-1 \times 10^{-3}$ à environ $1 \times 10^{-3}$, et B dans un intervalle s'étendant d'environ $-1 \times 10^{-4}$ à environ $1 \times 10^{-4}$.

**18.** Lentille LIO selon l'une quelconque des revendications 1 à 17, dans laquelle ladite lentille optique est formée d'un matériau souple biocompatible, dont un matériau acrylique souple, un hydrogel ou de la silicone.

**19.** Lentille LIO selon la revendication 18, dans laquelle ladite lentille optique présente une constante conique asphérique dans un intervalle s'étendant d'environ 0 à environ -50.

**20.** Lentille LIO selon la revendication 1, comprenant une lentille optique (12) ayant une surface antérieure réfractive (16) et une surface postérieure réfractive (18), au moins une desdites surfaces ayant une forme généralement torique présentant différentes valeurs de puissance optique le long de deux directions de surfaces orthogonales et présentant une asphéricité le long d'au moins une direction desdites directions orthogonales pour contrôler les aberrations d'un oeil modèle dans lequel la lentille LIO est implantée, de telle manière que la combinaison de la lentille et de l'oeil modèle présente une fonction de transfert de modulation d'au moins environ 0,25 et une profondeur de champ d'au moins environ 0,75 D pour un diamètre de pupille d'environ 4,5 mm et une longueur d'onde monochromatique d'environ 550 nm, telle que calculée dans l'oeil modèle.

**21.** Procédé de conception de lentille intraoculaire (10) ayant des surfaces réfractives antérieure et postérieure (16, 18), comprenant les étapes consistant à :

dériver une moyenne type des aberrations d'un oeil modèle en se basant sur des mesures de front d'onde d'aberrations présentées par les yeux d'une population de patients sélectionnée, et
ajuster l'asphéricité d'au moins une desdites surfaces réfractives pour contrôler lesdites aberrations moyennes de telle manière qu'un oeil modèle dans lequel la lentille est implantée présente un contraste d'image **caractérisé par** un contraste de fonction de transfert de modulation (FTM) maximum d'au moins environ 0,25 et une profondeur de champ d'au moins environ 0,75 D pour des diamètres de pupilles s'étendant de 4,5 mm à 5 mm, pour une lumière monochromatique d'une longueur d'onde d'environ 550 nm.

**Fig. 1A**

**Fig. 1B**

**Fig. 1C**

Fig. 2

## Fig. 3A

## Fig. 3B

## Fig. 4A

## Fig. 4B

## Fig. 5

## Fig. 6

**Fig. 7A**

**Fig. 7B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4504982 A, Burk
- WO 0189424 A, Pharmacia Groningen B.V.
- US 5922821 A
- US 6353069 B
- US 6786603 B
- US 20020105617 A